# EUROPEAN PATENT APPLICATION

(11) **EP 4 480 949 A1**
(43) Date of publication of application: **25.12.2024**
(21) Application number: 23774036.0
(22) Date of filing: 27.03.2023
(51) Int. Cl.: C07D 401/04, A61P 35/00, A61K 31/444

(54) **CRYSTAL FORM OF 1H-PYRROLO[2,3-C]PYRIDINE COMPOUND AND PREPARATION METHOD THEREFOR**

(30) Priority: 25.03.2022 CN 202210307050
(71) Applicant: Jumbo Drug Bank Co., Ltd., High-tech Zone Chengdu Sichuan 610000 (CN)
(72) Inventor: QIAN, Wenyuan, Shanghai 200131 (CN); WEI, Xiawei, Chengdu, Sichuan 610041 (CN); YANG, Chundao, Shanghai 200131 (CN); XU, Guanghai, Shanghai 200131 (CN); JIANG, Ning, Chengdu, Sichuan 610000 (CN)
(74) Representative: HGF
(86) International application number: PCT/CN2023/084146
(87) International publication number: WO 2023/179793

(57) **Abstract**

A crystal form of a 1H-pyrrolo[2,3-c]pyridine compound and a preparation method therefor. Also provided is a use of compound (II) and a crystal form thereof in the preparation of a drug for treating related diseases.

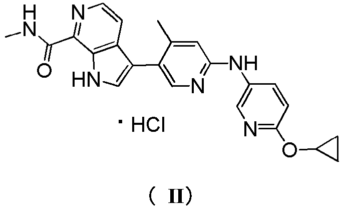

## Description

### The present application claims the following priority:

Application number: CN202210307050.9, and application date: March 25, 2022.

### TECHNICAL FIELD

The present invention relates to a crystal form of a 1H-pyrrolo[2,3-c]pyridine compound and a preparation method therefor. Also provided is a use of compound and a crystal form thereof in the preparation of a drug for treating related diseases.

### BACKGROUND

Colony stimulating factor 1 (CSF-1, also known as macrophage colony stimulating factor, M-CSF) is an important growth factor that controls the growth of myeloid progenitor cells, monocytes, macrophages, as well as osteoclasts and dendritic cells differentiated from macrophages. It must bind to its unique cell surface receptor CSF-1R to play its biological effect. CSF-1R is encoded by the proto oncogene c-FMS, so it is also called c-FMS, which is a receptor tyrosine kinase. The binding of CSF-1 and CSF-1R in the extracellular domain induces the dimerization of CSF-1R, which further leads to the self-phosphorylation of the intracellular CSF-1R kinase region. Once phosphorylation occurs, CSF-1R serves as a docking site of several cytoplasmic signaling molecules, and eventually triggers a series of signal cascade reactions. For example, the phosphorylation of tyrosine residue 697 of CSF-1R can activate MAPK signaling pathway, while the phosphorylation of tyrosine residue 721 of CSF-1R can activate PI3K and PLC γ signaling pathway.

Colony stimulating factor-1 receptor (CSF-1R) is a key target for regulating tumor associated macrophages in the tumor microenvironment. Many tumor cells can secrete growth factors such as CSF-1 during the growth process, and the latter can recruit macrophages (tumor associated macrophages, TAM) to enter the tumor area. Macrophages and tumor cells can also secrete CSF-1, their participation promotes the formation of a complex tumor microenvironment, which can help tumor cells produce immune tolerance to autoimmune function, and promote the proliferation, invasion and metastasis of tumor cells in vivo. Blocking the CSF-1/CSF1R pathway has been proved to significantly reduce the infiltration of macrophages in tumor sites, slow down the growth of primary tumors and reduce tumor metastasis. Therefore, inhibiting the survival/activation of macrophages by inhibiting CSF-1/CSF1R signaling has become an important strategy of cancer immunotherapy.

Recent studies have shown that the inhibitors of CSF-1R can be used in the field of disease treatment in various ways. It can be used alone or in combination with a variety of anti-cancer therapies, such as anti-angiogenesis, adoptive T cell cell transfer, radiotherapy, chemotherapy and immune checkpoint therapy. Many marketed drugs have the inhibitory activity of CSF-1R, such as imatinib, dasatinib and sunitinib, but there are no selective CSF-1R inhibitors on the market. Pexidartinib (PLX-3397), developed by Plexxikon and acquired by Daiichi Sankyo, is a dual inhibitor of CSF-1R and c-Kit. It has been approved by FDA in August 2019 for the treatment of tenosynovial giant cell tumor (TGCT).

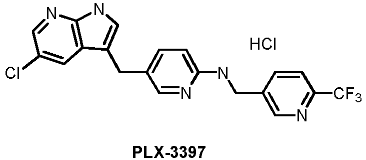

### SUMMARY OF THE INVENTION

The present application provides a crystal form A of a compound of formula (I), wherein the X-ray powder diffraction pattern thereof has characteristic diffraction peaks at 2Θ diffraction angles of 6.12±0.20°, 9.25±0.20° and 14.45±0.20°,

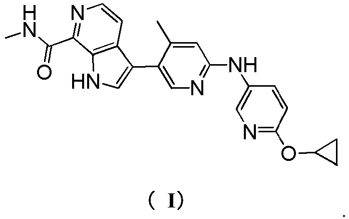

In some embodiments of the present application, the X-ray powder diffraction pattern of the above-mentioned crystal form A has characteristic diffraction peaks at 2Θ diffraction angles of 6.12 ± 0.20°, 7.98 ± 0.20°, 9.25 ± 0.20°, 14.45 ± 0.20°, 16.02 ± 0.20° and 24.52 ± 0.20°.

In some embodiments of the present application, the X-ray powder diffraction pattern of the above-mentioned crystal form A has characteristic diffraction peaks at 2Θ diffraction angles of 6.12±0.20°, 7.98±0.20°, 9.25±0.20°, 12.21±0.20°, 14.45±0.20°, 16.02±0.20°, 20.20±0.20° and 24.52±0.20°.

In some embodiments of the present application, the X-ray powder diffraction pattern of the above-mentioned crystal form A has characteristic diffraction peaks at 2Θ diffraction angles of 6.12±0.20°, 7.98±0.20°, 9.25±0.20°, 10.08±0.20°, 12.21±0.20°, 14.45±0.20°, 15.13±0.20°, 16.02±0.20°, 20.20±0.20°, 21.35±0.20°, 21.96±0.20° and 24.52±0.20°.

In some embodiments of the present application, the X-ray powder diffraction pattern of the above-mentioned crystal form A has characteristic diffraction peaks at 2Θ diffraction angles of 6.12°, 7.98°, 9.25°, 10.08°, 12.21°, 14.45°, 15.13°, 16.02°, 18.34°, 20.20°, 21.35°, 21.96°, 23.41°, 24.52°, 25.43°, 27.39°, 28.56°, 29.03°, 29.96°, 31.75° and 37.38°.

In some embodiments of the present application, the X-ray powder diffraction pattern of the above-mentioned crystal form A has characteristic diffraction peaks at 2Θ diffraction angles of 6.12±0.20°, 9.25±0.20°, and/or 14.45±0.20°, and/or 7.98±0.20°, and/or 16.02±0.20°, and/or 24.52±0.20°, and/or 12.21±0.20°, and/or 20.20±0.20°, and/or 10.08±0.20°, and/or 15.13±0.20°, and/or 18.34±0.20°, and/or 21.35±0.20°, and/or 21.96±0.20°, and/or 23.41±0.20°, and/or 25.43±0.20°, and/or 27.39±0.20°, and/or 28.56±0.20°, and/or 29.03±0.20°, and/or 29.96±0.20°, and/or 31.75±0.20°, and/or 37.38±0.20°.

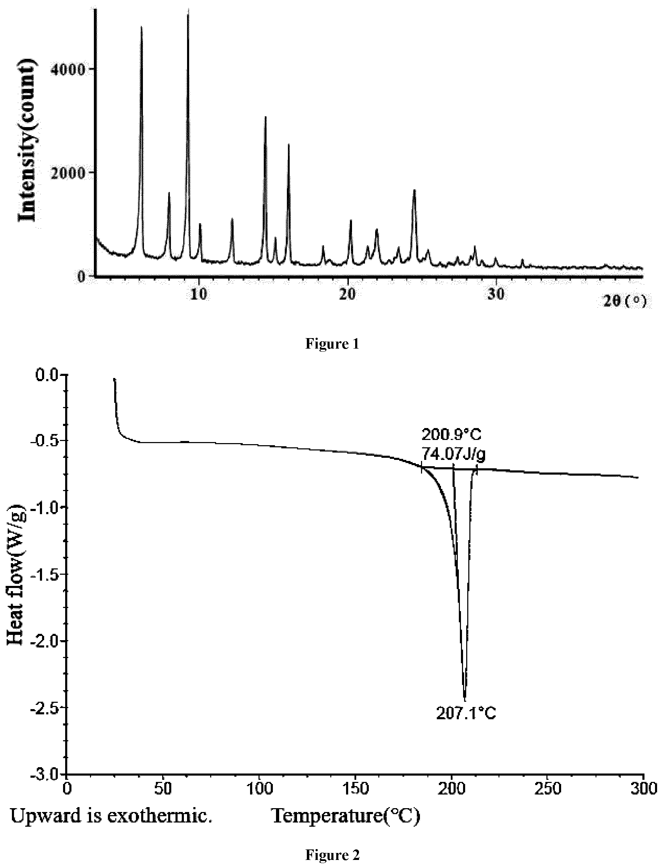

In some embodiments of the present application, the above-mentioned crystal form A has an X-ray powder diffraction (XRPD) pattern substantially as shown in figure 1.

In some embodiments of the present application, the XRPD pattern analysis data of the above-mentioned crystal form A are shown in Table 1.

**Table 1 XRPD pattern analysis data of crystal form A of the compound of formula (II)**

| **Number** | **2θ angles (°)** | **Interplanar distance (Å)** | **Intensity (count)** | **Relative intensity (%)** | **Number** | **2θ angles (°)** | **Interplanar distance (Å)** | **Intensity (count)** | **Relative intensity (%)** |
|---|---|---|---|---|---|---|---|---|---|
| 1 | 6.12 | 14.45 | 4457.92 | 91.93 | 12 | 21.96 | 4.05 | 716.76 | 14.78 |
| 2 | 7.98 | 11.08 | 1275.90 | 26.31 | 13 | 23.41 | 3.80 | 360.94 | 7.44 |
| 3 | 9.25 | 9.56 | 4849.32 | 100.00 | 14 | 24.52 | 3.63 | 1462.96 | 30.17 |
| 4 | 10.08 | 8.78 | 733.05 | 15.12 | 15 | 25.43 | 3.50 | 317.67 | 6.55 |
| 5 | 12.21 | 7.25 | 859.00 | 17.71 | 16 | 27.39 | 3.26 | 172.23 | 3.55 |
| 6 | 14.45 | 6.13 | 2912.10 | 60.05 | 17 | 28.56 | 3.13 | 373.13 | 7.69 |
| 7 | 15.13 | 5.86 | 530.06 | 10.93 | 18 | 29.03 | 3.08 | 122.05 | 2.52 |
| 8 | 16.02 | 5.53 | 2312.94 | 47.70 | 19 | 29.96 | 2.98 | 168.48 | 3.47 |
| 9 | 18.34 | 4.84 | 368.25 | 7.59 | 20 | 31.75 | 2.82 | 159.71 | 3.29 |
| 10 | 20.20 | 4.40 | 877.14 | 18.09 | 21 | 37.38 | 2.41 | 52.39 | 1.08 |
| 11 | 21.35 | 4.16 | 378.68 | 7.81 | | | | | |

In some embodiments of the present application, the above-mentioned crystal form A has a differential scanning calorimetry curve that has an endothermic peak with an onset of 200.9±3.0°C.

In some embodiments of the present application, the crystal form A has a differential scanning calorimetry (DSC) curve as shown in figure 2.

The present application also provides a compound of formula (II),

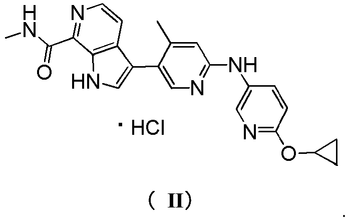

The present application provides a crystal form B of the compound of formula (II), wherein the X-ray powder diffraction pattern thereof has characteristic diffraction peaks at 2Θ diffraction angles of 4.579±0.200°, 6.898±0.200° and 15.918±0.200°,

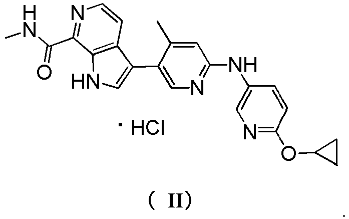

In some embodiments of the present application, the X-ray powder diffraction pattern of the above-mentioned crystal form B has characteristic diffraction peaks at 2Θ diffraction angles of 4.579±0.200°, 6.898±0.200°, 13.019±0.200°, 15.918±0.200°, 19.697±0.200° and 25.200±0.200°.

In some embodiments of the present application, the X-ray powder diffraction pattern of the above-mentioned crystal form B has characteristic diffraction peaks at 2Θ diffraction angles of 4.579±0.200°, 6.898±0.200°, 7.941±0.200°, 11.218±0.200°, 13.019±0.200°, 15.918±0.200°, 19.697±0.200° and 25.200±0.200°.

In some embodiments of the present application, the X-ray powder diffraction pattern of the above-mentioned crystal form B has characteristic diffraction peaks at 2Θ diffraction angles of 4.579±0.200°, 6.898±0.200°, 7.941±0.200°, 11.218±0.200°, 13.019±0.200°, 14.701±0.200°, 15.918±0.200°, 17.338±0.200°, 19.697±0.200°, 22.540±0.200°, 25.200±0.200° and 27.798±0.200°.

In some embodiments of the present application, the X-ray powder diffraction pattern of the above-mentioned crystal form B has characteristic diffraction peaks at 2Θ diffraction angles of 4.579±0.200°, 6.898±0.200°, and/or 15.918±0.200°, and/or 13.019±0.200°, and/or 19.697±0.200°, and/or 25.200±0.200°, and/or 7.941±0.200°, and/or 11.218±0.200°, and/or 9.070±0.200°, and/or 9.423±0.200°, and/or 13.796±0.200°, and/or 14.701±0.200°, and/or 15.182±0.200°, and/or 17.338±0.200°, and/or 18.939±0.200°, and/or 19.461±0.200°, and/or 20.700±0.200°, and/or 21.602±0.200°, and/or 22.200±0.200°, and/or 22.540±0.200°, and/or 22.818±0.200°, and/or 23.919±0.200°, and/or 24.461±0.200°, and/or 25.639±0.200°, and/or 26.563±0.200°, and/or 27.120±0.200°, and/or 27.798±0.200°, and/or 28.156±0.200°, and/or 28.595±0.200°, and/or 29.000±0.200°, and/or 29.339±0.200°, and/or 30.739±0.200°, and/or 32.064±0.200°, and/or 32.355±0.200°, and/or 33.201±0.200°, and/or 34.156±0.200°, and/or 35.020±0.200°, and/or 36.063±0.200°, and/or 39.176±0.200°.

In some embodiments of the present application, the X-ray powder diffraction pattern of the above-mentioned crystal form B has characteristic diffraction peaks at 2Θ diffraction angles of 4.579°, 6.898°, 7.941°, 9.070°, 9.423°, 11.218°, 13.019°, 13.796°, 14.701°, 15.182°, 15.918°, 17.338°, 18.939°, 19.461°, 19.697°, 20.700°, 21.602°, 22.200°, 22.540°, 22.818°, 23.919°, 24.461°, 25.200°, 25.639°, 26.563°, 27.120°, 27.798°, 28.156°, 28.595°, 29.000°, 29.339°, 30.739°, 32.064°, 32.355°, 33.201°, 34.156°, 35.020°, 36.063° and 39.176°.

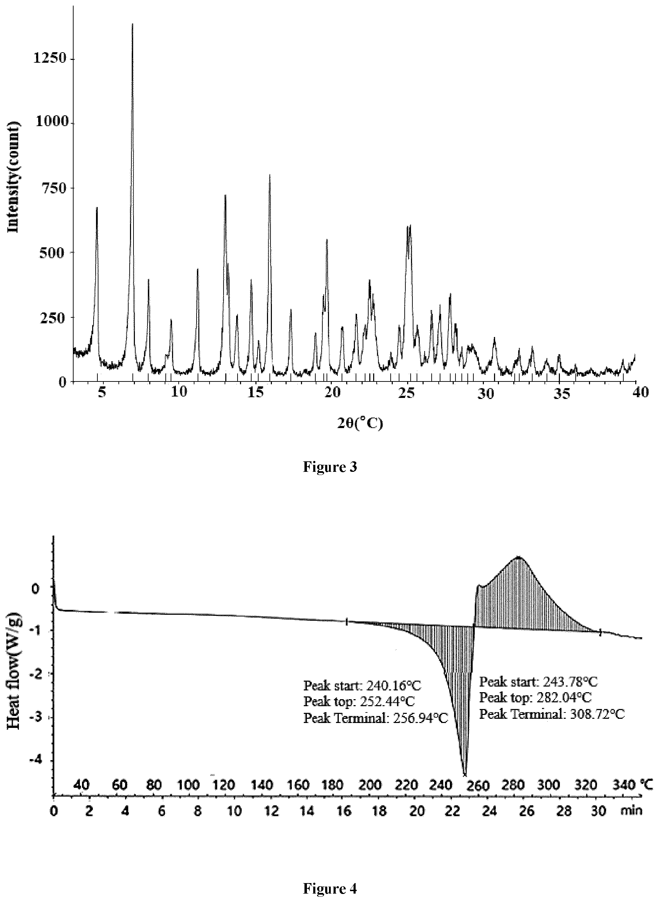

In some embodiments of the present application, the above-mentioned crystal form B has an XRPD pattern substantially as shown in figure 3.

In some embodiments of the present application, the XRPD pattern analysis data of the above-mentioned crystal form B are shown in Table 2.

**Table 2 XRPD pattern analysis data of crystal form B of the compound of formula (II)**

| **Number** | **2θ angles (°)** | **Interplanar distance (Å)** | **Intensity (count)** | **Relative intensity (%)** | **Number** | **2θ angles (°)** | **Interplanar distance (Å)** | **Intensity (count)** | **Relative intensity (%)** |
|---|---|---|---|---|---|---|---|---|---|
| 1 | 4.579 | 19.2808 | 617 | 46.2 | 21 | 23.919 | 3.7173 | 58 | 4.4 |
| 2 | 6.898 | 12.8041 | 1336 | 100.0 | 22 | 24.461 | 3.6361 | 167 | 12.5 |
| 3 | 7.941 | 11.1246 | 350 | 26.2 | 23 | 25.200 | 3.5312 | 540 | 40.4 |
| 4 | 9.070 | 9.7427 | 67 | 5.0 | 24 | 25.639 | 3.4716 | 146 | 10.9 |
| 5 | 9.423 | 9.3783 | 206 | 15.5 | 25 | 26.563 | 3.3530 | 206 | 15.4 |
| 6 | 11.218 | 7.8813 | 404 | 30.2 | 26 | 27.120 | 3.2853 | 220 | 16.5 |
| 7 | 13.019 | 6.7948 | 692 | 51.8 | 27 | 27.798 | 3.2068 | 273 | 20.5 |
| 8 | 13.796 | 6.4135 | 225 | 16.8 | 28 | 28.156 | 3.1667 | 157 | 11.7 |
| 9 | 14.701 | 6.0209 | 356 | 26.7 | 29 | 28.595 | 3.1191 | 64 | 4.8 |
| 10 | 15.182 | 5.8313 | 118 | 8.8 | 30 | 29.000 | 3.0765 | 104 | 7.8 |
| 11 | 15.918 | 5.5631 | 775 | 58.0 | 31 | 29.339 | 3.0417 | 101 | 7.5 |
| 12 | 17.338 | 5.1106 | 252 | 18.9 | 32 | 30.739 | 2.9063 | 137 | 10.3 |
| 13 | 18.939 | 4.6820 | 161 | 12.0 | 33 | 32.064 | 2.7891 | 49 | 3.7 |
| 14 | 19.461 | 4.5576 | 295 | 22.1 | 34 | 32.355 | 2.7648 | 94 | 7.1 |
| 15 | 19.697 | 4.5034 | 508 | 38.0 | 35 | 33.201 | 2.6962 | 107 | 8.0 |
| 16 | 20.700 | 4.2875 | 176 | 13.1 | 36 | 34.156 | 2.6230 | 58 | 4.3 |
| 17 | 21.602 | 4.1104 | 221 | 16.5 | 37 | 35.020 | 2.5602 | 70 | 5.3 |
| 18 | 22.200 | 4.0011 | 167 | 12.5 | 38 | 36.063 | 2.4885 | 33 | 2.5 |
| 19 | 22.540 | 3.9415 | 344 | 25.8 | 39 | 39.176 | 2.2976 | 51 | 3.8 |
| 20 | 22.818 | 3.8941 | 253 | 18.9 | | | | | |

In some embodiments of the present application, the above-mentioned crystal form B has a differential scanning calorimetry curve with an endothermic peak with an onset of 240.16±3.0°C and an exothermic peak having a peak top at 282.04±3.0°C.

In some embodiments of the present application, the above-mentioned crystal form B has a differential scanning calorimetry curve as shown in figure 4.

In some embodiments of the present application, the above-mentioned crystal form B has a weight loss of 0.3643% at 200.0 ± 3°C in its thermo gravimetric analysis curve.

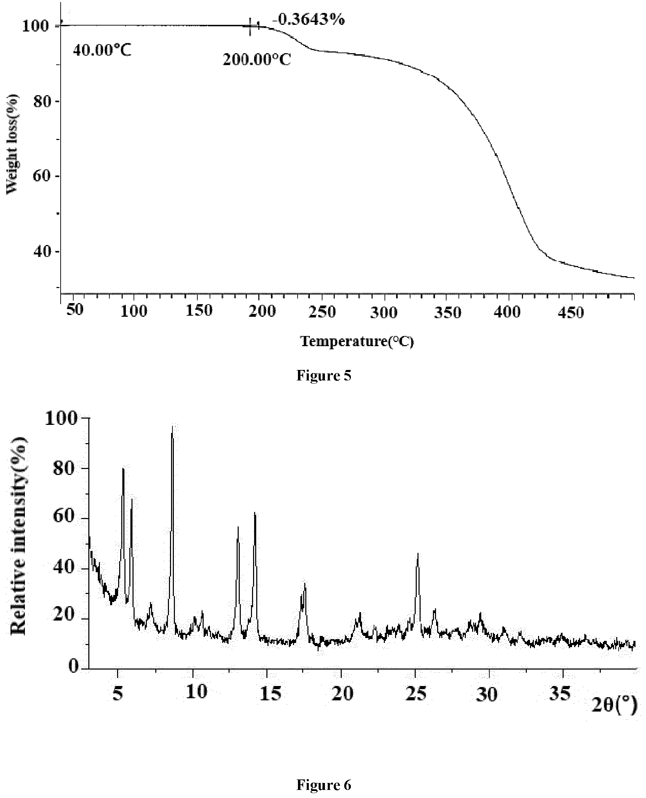

In some embodiments of the present application, the above-mentioned crystal form B has a thermo gravimetric analysis curve as shown in figure 5.

The present application provides a crystal form C of the compound of formula (III), wherein the X-ray powder diffraction pattern thereof has characteristic diffraction peaks at 2Θ diffraction angles of 5.35±0.20°, 8.66±0.20° and 14.22±0.20°,

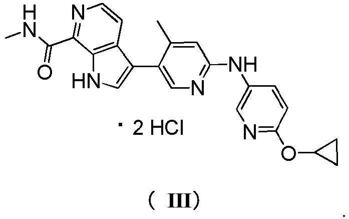

In some embodiments of the present application, the X-ray powder diffraction pattern of the above-mentioned crystal form C has characteristic diffraction peaks at 2Θ diffraction angles 5.35±0.20°, 5.91±0.20°, 8.66±0.20°, 13.12±0.20°, 14.22±0.20° and 25.19±0.20°.

In some embodiments of the present application, the X-ray powder diffraction pattern of the above-mentioned crystal form C has characteristic diffraction peaks at 2Θ diffraction angles of 5.35±0.20°, 5.91±0.20°, 8.66±0.20°, 13.12±0.20°, 14.22±0.20°, 17.61±0.20°, 25.19±0.20° and 26.34±0.20°.

In some embodiments of the present application, the X-ray powder diffraction pattern of the above-mentioned crystal form C has characteristic diffraction peaks at 2Θ diffraction angles of 5.35±0.20°, 5.91±0.20°, and/or 8.66±0.20°, and/or 13.12±0.20°, and/or 14.22±0.20°, and/or 17.61±0.20°, and/or 25.19±0.20°, and/or 26.34±0.20°, and/or 7.17±0.20°, and/or 10.63±0.20°, and/or 17.34±0.20°, and/or 21.22±0.20°, and/or 22.28±0.20°, and/or 29.39±0.20°, and/or 31.03±0.20°, and/or 32.10±0.20°, and/or 34.91±0.20°.

In some embodiments of the present application, the X-ray powder diffraction pattern of the above-mentioned crystal form C has characteristic diffraction peaks at 2Θ diffraction angles of 5.35°, 5.91°, 7.17°, 8.66°, 10.63°, 13.12°, 14.22°, 17.34°, 17.61°, 21.22°, 22.28°, 25.19°, 26.34°, 29.39°, 31.03°, 32.10°and 34.91°.

In some embodiments of the present application, the above-mentioned crystal form C has an XRPD pattern substantially as shown in figure 6.

In some embodiments of the present application, the XRPD pattern analysis data of the above-mentioned crystal form C are shown in Table 3.

**Table 3 XRPD pattern analysis data of crystal form C of the compound of formula (III)**

| **Number** | **2θ angles (°)** | **Interplanar distance (Å)** | **Intensity (count)** | **Relative intensity (%)** | **Number** | **2θ angles (°)** | **Interplanar distance (Å)** | **Intensity (count)** | **Relative intensity (%)** |
|---|---|---|---|---|---|---|---|---|---|
| 1 | 5.35 | 16.51 | 823.45 | 67.28 | 10 | 21.22 | 4.19 | 111.11 | 9.08 |
| 2 | 5.91 | 14.95 | 667.36 | 54.52 | 11 | 22.28 | 3.99 | 67.45 | 5.51 |
| 3 | 7.17 | 12.34 | 107.59 | 8.79 | 12 | 25.19 | 3.54 | 456.83 | 37.32 |
| 4 | 8.66 | 10.21 | 1223.96 | 100.00 | 13 | 26.34 | 3.38 | 147.26 | 12.03 |
| 5 | 10.63 | 8.32 | 140.19 | 11.45 | 14 | 29.39 | 3.04 | 142.75 | 11.66 |
| 6 | 13.12 | 6.75 | 648.33 | 52.97 | 15 | 31.03 | 2.88 | 61.15 | 5.00 |
| 7 | 14.22 | 6.23 | 758.55 | 61.98 | 16 | 32.10 | 2.79 | 52.92 | 4.32 |
| 8 | 17.34 | 5.12 | 233.98 | 19.12 | 17 | 34.91 | 2.57 | 43.40 | 3.55 |
| 9 | 17.61 | 5.04 | 347.98 | 28.43 | | | | | |

In some embodiments of the present application, the crystal form C has a differential scanning calorimetry curve with an endothermic peak with an onset of 223.2±3.0°C.

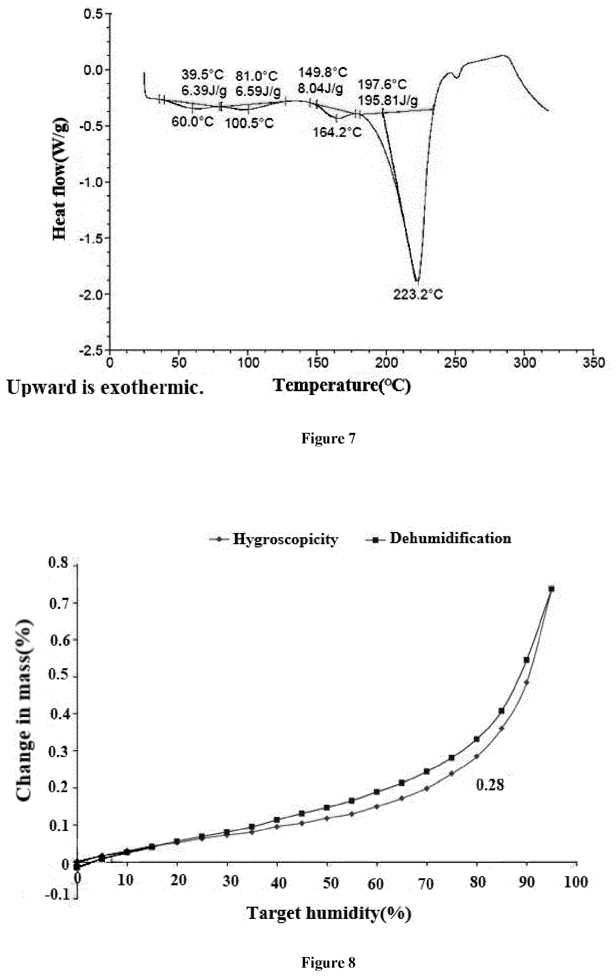

In some embodiments of the present application, the DSC pattern of the above-mentioned crystal form C has a differential scanning calorimetry curve as shown in figure 7.

The present application also provides a method for preparing the crystal form B of the compound of formula (II), wherein the method comprises:
1) adding compound of formula (I) to a solvent to form a solution; and
2) adding 12N concentrated hydrochloric acid to the solution, stirring at a certain temperature for a certain time, filtering, and drying the filter cake in a vacuum;
wherein,
the solvent is alcohols solvent;
the stirring temperature is 30°C to 50°C
and the stirring time is 12 hours to 24 hours.

The present application also provides a method for preparing the crystal form B of the compound of formula (II), wherein the method comprises:
1) adding compound of formula (I) to a solvent to form a solution; and
2) adding 12N concentrated hydrochloric acid to the solution, stirring at a certain temperature for a certain time, filtering, and drying the filter cake in a vacuum;
wherein,
the solvent is alcohols solvent;
the stirring temperature is 40°C to 45°C
and the stirring time is 12 hours to 16 hours.

The present application also provides use of crystal form A, crystal form B or crystal form C of the above-mentioned compound of formula (II) in the preparation of a medicament related for treating tenosynovial giant cell tumor.

### Definition and description

Unless otherwise specified, the following terms and phrases used herein are intended to have the following meanings. A specific phrase or term should not be regarded as uncertain or unclear without a specific definition, but should be understood according to the common meaning. When a trade name appears in the present application, it is intended to refer to its corresponding product or its active ingredient.

The intermediate compound of the present application can be prepared by a variety of synthesis methods familiar to those skilled in the art, including the detailed embodiments listed below, the detailed embodiments formed by its combination with other chemical synthesis methods, and the equivalent replacement methods familiar to those skilled in the art. The preferred embodiments includes but not limited to the examples of the present application.

The chemical reaction of the detailed embodiments of the present application is completed in a suitable solvent, which must be suitable for the chemical changes of the present application and the required reagents and materials. In order to obtain the compound of the present application, it is sometimes necessary for those skilled in the art to modify or select the synthesis step or reaction process on the basis of the existing embodiments.

The structure of the compound of the present application can be confirmed by the conventional method familiar to those skilled in the art. If the present application relates to the absolute configuration of the compound, the absolute configuration can be confirmed by the conventional technical means in the art. For example, the single crystal X-ray diffraction (SXRD) method collects the diffraction intensity data of the cultured single crystal with Bruker D8 venture diffractometer, the light source is CuKα radiation and the scanning method is ϕ/ω scanning. After collecting the relevant data, the crystal structure is further analyzed by the direct method (Shelxs97), and the absolute configuration can be confirmed.

The present application will be described in detail through the following examples, which do not imply any limitations on the present application.

All solvents used in the present application are commercially available and can be used without further purification.

The solvent used in the present application can be commercially available.

Compounds are named according to the conventional naming principles in the art or using the ChemDraw^{®} software and the commercial compounds are named by supplier directory.

### Technical effect

The compound of the present application has stable crystal form and slight hygroscopicity; and the crystal form of the compound of the present application can significantly inhibit the proliferation of BCR-FMS-BaF3 cells.

### X-ray powder diffraction (X-ray powder diffractometer, XRPD) method of the present application

Crystal form A of compound of formula (I) and crystal form C of compound of formula (III):
Instrument name: PANalytical X'pert³ X-ray diffractometer,
Test method: About 10 mg of sample was evenly laid on the monocrystalline silicon sample plate.

**Table 4 XRPD experimental parameters**

| | |
|---|---|
| Step size: 0.0263 degree | Scanning time per step: 46.665 seconds |
| Kα1=1.54060 Å | Kα2=1.54443 Å |
| Light tube voltage: 45 kV, light tube current: 40 mA | Scanning angle range: 3-40 deg |

Crystal form B of compound of formula (II):
Instrument name: X-ray diffractometer
Instrument model: DX-2700BH
Instrument manufacturer: Dandong Haoyuan Instrument Co., Ltd

Method parameters:
Light tube: Cu, k-Alphal (λ=1.54184 Å)
Light tube voltage: 40 kV, light tube current: 30 Ma
Divergence slit: 1mm
Detector slit: 0.3 mm
Anti-scattering slit: 1 mm
Scanning range: 3-40 deg
Step diameter: 0.02 deg
Step size: 0.5 seconds

Test method: The sample was taken and placed on a sample plate to ensure that the surface of the sample plate is flat. Finally, the sample plate was placed in an X-ray diffractometer for testing.

### Differential Scanning Calorimetry (Differential Scanning Calorimeter, DSC) method of the present application

Crystal form A of compound of formula (I) and crystal form C of compound of formula (III):
Instrument model: TA2500 differential scanning calorimeter

**Table 5 DSC Experiment Parameter**

| Sample plate | Aluminum plate, gland |
|---|---|
| Temperature range /°C | 25-300 |
| Scan rate/°C /min | 10 |
| Protective gas | Nitrogen |

Crystal form B of compound of formula (II):
Instrument model: Mettler Toledo DSC 1 Differential scanning calorimeter
Test method: The sample (2.44 mg) was taken and placed in a DSC high-pressure crucible, tested after pressed and sealed and heated from 40°C to 350°C at a heating rate of 10°C/min.

### Thermal gravimetric analyzer (TGA) method of the present application

Instrument model: TGA2SF/1100
Test method: The sample (2.745mg) was taken and placed in an alumina crucible for testing, and the sample was heated from 40°C to 500°C at a heating rate of 10°C/min.

### Dynamic vapor sorption (DVS) method of the present application

Instrument model: SMS DVS Intrinsic dynamic vapor sorption instrument
Test conditions: The sample (10 mg) was taken and placed in a DVS sample plate for testing.
Detailed DVS parameters were as follows:
Temperature: 25°C
Balance: dm/dt=0.01%/min
Drying: 25°C, 0% RH drying for 2hours
Rh (%) test step: 5% RH
Range of Rh (%) test step: 0% -95% -0% RH

**Table 6 Evaluation and classification of hygroscopicity**

| **Classification of hygroscopicity** | ΔW% |
|---|---|
| Deliquesce | absorb enough water to form liquid |
| High hygroscopicity | ΔW% ≥ 15% |
| General hygroscopicity | 15% > ΔW% ≥ 2% |
| Slight hygroscopicity | 2% > ΔW% ≥ 0.2% |
| No or little hygroscopicity | ΔW% < 0.2% |

| | |
|---|---|
| Note: ΔW% represents the hygroscopic weight gain of the test sample at 25°C±1°C and 80%±2% RH. | |

### BRIEF DESCRIPTION OF THE DRAWINGS

Figure 1 shows an X-ray powder diffraction (XRPD) pattern using Cu-Kα radiation of compound A of formula (I);
Figure 2 shows a differential scanning calorimetry (DSC) curve of compound A of formula (I);
Figure 3 shows an XRPD pattern using Cu-Kα radiation of compound B of formula (II);
Figure 4 shows a DSC curve of compound B of formula (II);
Figure 5 shows a thermo gravimetric analysis (TGA) curve of compound B of formula (II);
Figure 6 shows an XRPD pattern using Cu-Kα radiation of compound C of formula (III);
Figure 7 shows a DSC pattern of compound C of formula (III);
Figure 8 shows a dynamic vapor sorption (DVS) curve of compound B of formula (II).

### DETAILED DESCRIPTION

In order to better understand the content of the present application, further explanations in combination with detailed examples are introduced as follows, without limiting the content of the present application.

### Example 1: Preparation of compound of formula (I)

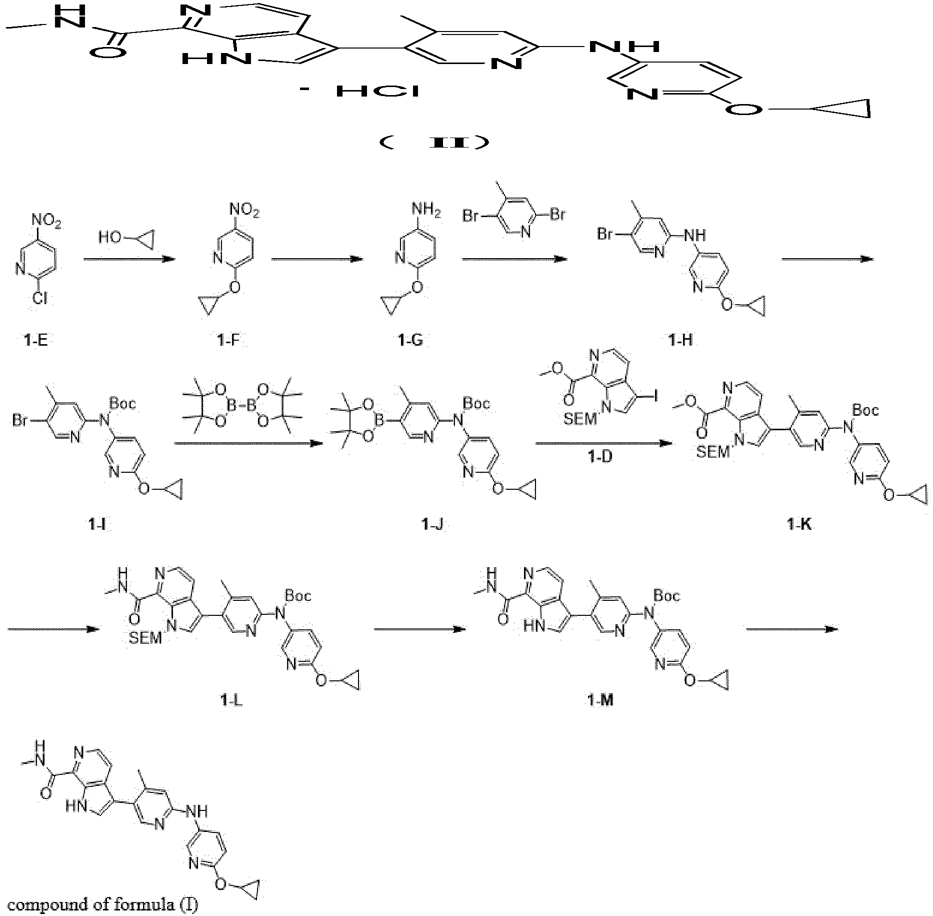

### Step 1: Synthesis of compound 1-B

Compound 1-A (125g, 819.24mmol) was dissolved in methanol (2.5L) and toluene (2.5L), 1,1'-bis (diphenylphosphino)ferrocene-palladium(II) dichloride dichloromethane complex (40g, 48.98mmol) and triethylamine(331.59g, 3.28mol) were added therein, then reaction was performed under stirring for 40 hours in a high-pressure autoclave with an atmosphere of carbon monoxide (2MPa) and an external temperature of 110°C. After the reaction was completed, the resulted was filtered with diatomite and concentrated under reduced pressure to remove the solvent to obtain a crude product. The crude product was purified by column chromatography (n-heptane: ethyl acetate=1:1) to obtain compound **1-B.**
MS *m*/*z*: 177.2 [M+H] ⁺

### Step 2: Synthesis of compound 1-C

Compound **1-B** (249.9g, 1.42mol) was dissolved in N,N-dimethylformamide (2.5L) to prepare a reaction solution. The reaction solution was cooled to a temperature of 0°C, N-iodosuccinimide (351.05g, 1.56mol) was added therein, and the reaction solution was stirred at 0°C to 5°C for 1 hour. The reaction solution was slowly poured into water (9L), stirred for 15 minutes and then filtered under reduced pressure to obtain a filter cake. The filter cake was washed twice with 4L of water. After the filter cake was dried in an oven under reduced pressure, ethanol (800ml) was added therein, stirred for 1 hour, and then filtered under reduced pressure to obtain compound **1-C.**
MS *m*/*z*: 302.7 [M+H] ⁺

### Step 3: Synthesis of compound 1-D

Compound **1-C** (200g, 662.10mmol) was dissolved in tetrahydrofuran (3L), the resulted reaction system was cooled to a temperature of 0°C to 5°C, sodium hydride (39.73g, 993.15mmol, 60% content) was slowly added in batches, followed by stirring at 0°C to 5°C for 0.5 hours, then 2-chloromethyl 2-(trimethylsilyl)ethyl ether (139.09g, 834.25 mmol) was added therein, and the resulted reaction system was naturally raised to a temperature of 20°C and stirred for 1 hour. Under the atmosphere of weak nitrogen flow, the reaction solution was slowly poured into 3 L of water, stirred and quenched. Ethyl acetate (3L) was added to perform extraction, and the resulted organic phase was washed with water (3L), and then washed with saturated salt solution (3L). The organic phase was dried with anhydrous Na₂SO₄, filtered, and concentrated under reduced pressure to remove the solvent to obtain a crude product. The crude product was purified by column chromatography (n-heptane: tetrahydrofuran=7:1 to 4:1) to obtain a purified crude product., then n-heptane (2L) was added to the purified crude product, stirred for 1 hour, and then filtered under reduced pressure to obtain compound **1-D.**
MS *m*/*z*: 433.1 [M+H] ⁺

### Step 4: Synthesis of compound 1-F

Compound **1-E** (2.5kg, 15.77mol) and cesium carbonate (10.28kg, 31.54mol) were dissolved in tetrahydrofuran (10L). The reaction system was cooled to a temperature of 0°C to 10°C, and cyclopropanol (1.14kg, 19.71mol) was added therein by dripping. After the dripping was completed, the reaction solution was slowly heated to a temperature of 25°C to 30°C and stirred for 16 hours. The reaction solution was filtered under reduced pressure to obtain a filter cake, the obtained filter cake was rinsed twice with tetrahydrofuran (1L), and the obtained filtrates were combined. The obtained filtrate was slowly added to water (36L), stirred for 30 minutes and then filtered under reduced pressure to obtain a filter cake. Water (5L) was added to the obtained filter cake, stirred for 30 minutes, and then filtered under reduced pressure to obtain compound **1-F.**
MS *m*/*z*: 180.8 [M+H] ⁺

### Step 5: Synthesis of compound 1-G

Compound **1-F** (100g, 555.06mmol) was dissolved in ethanol (1L), and Pd/C (5g, 555.06umol, 10% content) was added therein to perform reaction in a hydrogen atmosphere (30psi to 40psi), and stirred at 25°C to 30°C for 16 hours. The reaction solution was filtered by diatomite. Be careful not to drain the diatomite. The obtained filtrate was subjected to rotary evaporation under reduced pressure to remove the solvent to obtain compound **1-G.**
MS *m*/*z*: 151.2 [M+H] ⁺

### Step 6: Synthesis of compound 1-H

Compound **1-G** (154g, 1.03mol) was dissolved in 1,4-dioxane (2.25L) and water (45ml), and 2,5-dibromo-4-methylpyridine (308.77g, 1.23mol), 4,5-bis(diphenylphosphino)-9,9-dimethylxanthene (29.67g, 51.27mmol) and potassium carbonate (354.31g, 2.56mol) were added therein. The reaction system was replaced with nitrogen for three times, then tris(dibenzylidene acetone)dipalladium (14.74g, 25.64 mmol) was added therein, the reaction system was replaced with nitrogen for three times, and the reaction solution was stirred for 16 hours under nitrogen protection at 55°C to 60°C. The resulted reaction solution was filtered by diatomite to obtain a filter cake, and the filter cake was rinsed with 1,4-dioxane (500ml*3). The obtained filtrate (in a volume of about 6L) was concentrated under reduced pressure to a remaining volume of about 3.5L, and then was slowly added to n-heptane(17.5L) and stirred for 0.5 hours, and then filtered under reduced pressure to obtain a filter cake. Water (3L) was added therein, stirred for 20 minutes, and then filtered under reduced pressure to obtain compound **1-H.**
MS *m*/*z*: 319.9 [M+H] ⁺

### Step 7: Synthesis of compound 1-I

Compound 1-H (2.18kg, 6.80mol) was dissolved in dichloromethane (17.5L), 4-dimethylaminopyridine (41.56g, 340.21mmol) was added therein, and then di-tert butyl dicarbonate (1.93kg, 8.85mol) was added dropwise. The resulted reaction solution was stirred at an external temperature ranging from 30°C to 40°C for 3 hours. Water (10 L) was added to the reaction solution for washing. The organic phase was washed with saturated salt solution (10L), and the organic phase was collected, dried with anhydrous sodium sulfate (1kg), and concentrated under reduced pressure to obtain a crude product. Then n-heptane (5L) was added to the crude product, and stirring was performed with an ice bath at 0°C, so that 15 minutes later a solid was gradually precipitated. The stirring was continued for 1 hour and then the resulted was suction filtered to obtain compound **1-I**.
MS *m*/*z*: 420.1[M+H] ⁺

### Step 8: Synthesis of compound 1-J

Compound **1-I** (500.31g, 1.19 mol), bis(pinacolato)diboron (392.81g, 1.55mol), dioxane (5L) and potassium acetate (236.73g, 2.38 mol) were added in sequence into a reactor. The reaction system was replaced with nitrogen gas for three times, and 1,1'-bis(diphenylphosphino)ferrocene chloride palladium (43.71g, 0.08 mol) was added therein. The reaction system was replaced with nitrogen gas for three times, and nitrogen flow protection was turned on. The reaction system was heated to a temperature of 70°C to 80°C and stirred for 12 hours to 16 hours. The resulted reaction solution was suction filtered with diatomite (200g) under reduced pressure to obtain a filter cake. The filter cake was rinsed with ethyl acetate (1L*2), and the filtrates were combined and concentrated to obtain a crude product. The crude product was purified by column chromatography (eluent: n-heptane: tetrahydrofuran=10/1) to obtain compound **1-J.**
MS *m*/*z*: 490.3 [M+H+Na] ⁺

### Step 9: Synthesis of compound 1-K

Compound **1-D** (385.26g, 0.89mol), Compound **1-J** (466.95g, 0.91mol) and dioxane (3.85L) were added in sequence into a reactor and stirred. Potassium phosphate (385.81g, 1.82mol) was dissolved in water (385mL), and was added to the reactor at room temperature (20°C to 30°C). The reaction system was replaced with nitrogen gas for three times, and then cooled to a temperature of 0°C to 5°C. 1,1'-bis(diphenylphosphine)ferrocene palladium chloride dichloromethane complex (65.25g, 0.09mol) was added therein, and the reaction system was replaced with nitrogen gas for three times. The reaction system was heated to a temperature of 35°C to 40°C and stirred for 12 hours to 16 hours. Ethyl acetate (8L) and water (8L) were added to the reaction solution, followed by stirring for 10 minutes and then standing to allow liquid-liquid separation to obtain an aqueous phase and an organic phase. The aqueous phase was separated out. The organic phase was washed with water (8L) and saturated sodium chloride solution (8L). The organic phase was collected, dried with anhydrous sodium sulfate (500g), filtered under reduced pressure, and concentrated under reduced pressure to obtain a crude product. Ethyl acetate (7.7L) and powdered activated carbon (193.38g) were added to the crude product. The resulted was reflux stirred (at 75°C to 80°C) for 1 hour, then cooled to a temperature of 55°C to 60°C, and filtered with diatomite (500g) under reduced pressure to obtain a filter cake. The filter cake was rinsed with ethyl acetate (15L) to produce filtrates. The filtrates were combined and concentrated under reduced pressure (at a temperature not exceeding 45°C) to a volume of about 2L, then into which ethyl acetate (0.3L) and n-heptane (3L) were added, and the resulted was stirred at 20°C to 30°C for 12 hours to 16 hours, and filtered to obtain compound **1-K.**
MS *m*/*z*: 646.4[M+H] ⁺

### Step 10: Synthesis of compound 1-L

Ethanol (705ml) was added to a 3L reaction vessel, then compound **1-K** (352g, 0.55mol) was added therein and stirring was performed. Then 260mL of methylamine ethanol solution was added to the reaction vessel, and stirring was continued, obtaining a turbid reaction solution. The reaction solution was slowly heated to a temperature of 35°C to 40°C and stirred for 2 hours to 3 hours. Another 260ml of methylamine ethanol solution was added to the reaction vessel, and stirring was continued, obtaining a turbid reaction solution. The reaction solution was stirred at 35°C to 40°C for 20 hours to 24 hours, during which the reaction solution gradually became clear. The reaction solution was concentrated under reduced pressure to obtain a crude product **1-L** which was directly used for the next reaction without further purification.
MS *m*/*z*: 645.2[M+H] ⁺

### Step 11: Synthesis of compound 1-M

704ml tetrahydrofuran was added to the crude product **1-L,** and 1100ml tetrabutylammonium fluoride tetrahydrofuran solution was added to a reaction flask under stirring conditions, obtaining a clear reaction solution. Then 66ml of anhydrous ethylenediamine was added to the reaction flask. The reaction solution was stirred at 65°C to 70°C for 18 hours. The reaction solution was concentrated under reduced pressure (50°C) until no solvent was evaporated, then 2L ethyl acetate and 2L water were added for performing liquid-liquid extraction, and the organic phase was washed with water (2L*4). The organic phase was collected and concentrated under reduced pressure (50°C) until no solvent was evaporated. 700ml of water was added to the rotary evaporation flask, obtaining a turbid solution which was then transferred to a round bottom flask and stirred at 20°C to 30°C for 16 hours. The resulted was filtered under reduced pressure using a Buchner funnel to obtain a filter cake. The filter cake was rinsed with water (50ml*3) to obtain a solid. The solid was stirred again with acetonitrile (700ml) at 20°C to 30°C for 30 minutes. The resulted was filtered under reduced pressure using a Buchner funnel to obtain a filter cake. The filter cake was rinsed with acetonitrile (30ml*3) to obtain compound **1-M.**
MS *m*/*z*: 515.3[M+H] ⁺

### Step 12: Synthesis of compound of formula (I)

Ethyl acetate (3.4L) was added into a 10L three necked bottle. Compound **1-M** (168g) was added into the three necked bottle, and stirring was continued, obtaining a clear reaction solution. 800ml of hydrochloric acid/ethyl acetate (4mol/L) were slowly added to the reaction vessel, and solid precipitates from the reaction solution. The reaction system was gradually heated from 20°C to 30°C to 35°C to 40°C, and stirred for 19 hours under this condition. The reaction solution was filtered under reduced pressure to obtain a yellow solid which was rinsed with ethyl acetate (50ml*3). The yellow solid was added into 1700ml of water in a vessel under stirring, obtaining a clear reaction system. The reaction system was adjusted to a pH of 9 to 10 using a prepared 2.0L of NazCOs solution (1mol/L), light green solid was precipitated and stirring was continued for 0.5 hours to 1 hour. The light green solid was added into a round bottom flask containing acetonitrile (1.7L) and stirred at 20°C to 30°C for 0.5 hours to 1 hour to obtain a reaction mixture. The reaction mixture was filtered under reduced pressure to obtain the **compound of formula (I).**
MS *m*/*z*: 415.2[M+H] ⁺

### Example 2: Preparation of crystal form B of compound of formula (II)

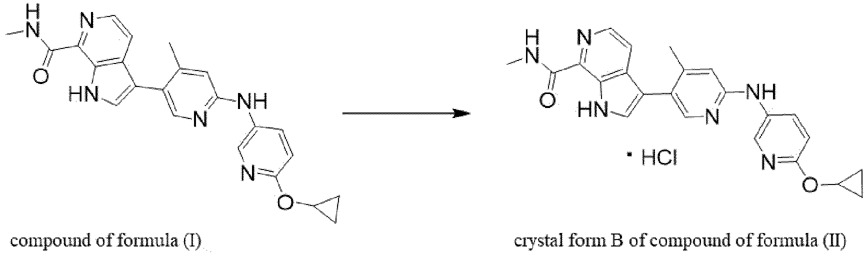

### Step 1: Synthesis of crystal form B of compound of formula (II)

EtOH (2.5L) was added into a 5L reaction vessel, then **compound of formula (I)** (121.2g) was added and stirred. 26ml of concentrated hydrochloric acid (12mol/L) was slowly added into the reaction vessel, and the reaction solution was dissolved first and then became turbid. The reaction system was gradually heated to a temperature of 40°C to 45°C and stirring was continued for 12 hours to 16 hours. Then the reaction system was naturally cooled to 10°C to 20°C, filtered under reduced pressure using a Buchner funnel to obtain a filter cake. The filter cake was washed with ethanol (50ml*3), and the filter cake was collected. The obtained filter cake was dried in a vacuum (45°C, -0.1Mpa, 12 hours) to obtain crystal form B of compound of formula (II). The compound was determined to be with one hydrochloride (Cl⁻% was 7.6%) by measuring IC-Cl.

MS *m*/*z*: 415.2[M+H] ⁺; 1HNMR (400MHz, DMSO-*d*₆) δ 12.22 (s, 1H), 10.63 (s, 1H), 9.07 (d, J=4.8 Hz, 1H), 8.37 (d, J=2.8 Hz, 1H), 8.24 (d, J=5.6 Hz, 1H),7.99 (s, 1H), 7.93-7.90 (m, 2H), 7.71 (d, J=5.6 Hz, 1H) 7.15 (s, 1H) 7.00 (d, J=8.8 Hz, 1H), 4.23-4.19(m, 1H) 2.91 (d, J=4.8 Hz,3H) 2.27(s, 3H) 0.79 - 0.67(m, 4H).

### Example 3: Preparation of crystal form A of compound of formula (I)

1.6g of crystal form B of compound of formula (II) was weighed and dissolved in 25 ml of water, and stirred at 10°C to 20°C, obtaining a solution. 1.88 g of sodium carbonate was dissolved in 5 ml of water and slowly added to the above solution, and stirring was continued for 0.5 hours. The reaction solution became from clear to turbid, and solid precipitation occurred. The solid was filtered. The filter cake was dried for 12 hours in a vacuum to obtain the crystal form A of compound of formula (I). MS *m*/*z:* 415.2[M+H] ⁺.

### Example 4: Preparation of crystal form C of compound of formula (III)

The crystal form A of compound of formula (I) (1.2g, 2.90mmol) was weighed and mixed with ethanol (18ml), and concentrated HCl (12M, 603.20uL, 2.5eq) was added therein, and solid dissolution was observed. The resulted was stirred in an oil bath at 50°C overnight, filtered and dried to obtain crystal form C of compound of formula (III), which was determined to be with two hydrochloride salts by measuring IC-CL (Cl⁻% was 18.03%).

### Example 5: Study on hygroscopicity of crystal form B of compound of formula (II)

Experimental materials:
   SMS DVS intrinsic dynamic vapor adsorption instrument
Experimental method:
   A sample (10 mg) was taken and placed in a DVS sample plate for testing.
Experimental results:
   DVS curve of crystal form B of compound of formula (II) was shown in figure 8, △W= 0.28%.
Experimental conclusion:
   The crystal form B of compound of formula (II) has a hygroscopic weight gain of 0.28% at 80%RH/25°C, indicating the sample had a slight hygroscopicity.

### Example 6: Solid stability test of crystal form B of compound of formula (II)

About 900mg of crystal form B of compound of formula (II) was respectively weighed, placed at the bottom of a glass sample bottle, spread into a thin layer so that and the samples were placed for full exposure. The samples placed at high temperature and humidity were sampled for XRPD testing on the 10th and 30th day; The samples exposed to light were put into a clean surface dish, laid into a thin layer, covered with a quartz glass cover, and sampled for XRPD testing on the 5th and 10th days; For the long-term test and accelerated test (shading), each sample were put into a double-layer LDPE bag, each layer of LDPE bag was respectively buckled and sealed, and then the LDPE bag was put into an aluminum foil bag with a package of desiccant and heat sealed. The samples were respectively placed at 25°C/60% RH, 30°C/65% RH and 40°C/75% RH for exploration, the test results were compared with the initial test results of 0 day and the test results were shown in Table 7 below.

**Table 7 Solid stability test results of crystal form B of compound of formula (II)**

| Test condition | Point of time | Crystal form |
|---|---|---|
| - | 0 days | crystal form B |
| high temperature(60°C, open) | 10 days | crystal form B |
| | 30 days | crystal form B |
| high humidity (25°C/relative humidity 92.5%, open) | 10 days | crystal form B |
| | 30 days | crystal form B |
| strong light (5000 lx and UV intensity 90w/cm², open) | 5 days | crystal form B |
| | 10 days | crystal form B |
| 25°C/60%RH | 3M | crystal form B |
| | 6M | crystal form B |
| | 24M | crystal form B |
| | 24M | crystal form B |
| 30°C/65%RH | 1M | crystal form B |
| | 3M | crystal form B |
| | 6M | crystal form B |
| 40°C/75%RH(open) | 1M | crystal form B |
| | 3M | crystal form B |
| | 6M | crystal form B |

| | | |
|---|---|---|
| **Conclusion:** The crystal form B of compound of formula (II) had good stability under influencing factors, light, acceleration and long-term experimental conditions. | | |

### Biological test data:

Luminescent Cell Viability Assay (CellTiter-Glo^{®} methold) from Promega was used for cell proliferation detection. CellTiter-Glo^{®} Luminescent Cell Viability Assay (CellTiter-Glo^{®} luminescent cell viability detection kit) was a homogeneous rapid detection method for detecting the viability of living cells in culture by quantitative determination of ATP. ATP was an indicator of the metabolism of living cells. Cell lysis and the resulting light signal were proportional to the amount of present ATP, and the amount of ATP is directly proportional to the number of living cells in the culture. The higher the fluorescence reading indicated the higher ATP content in the cell and also the higher the cell viability. Therefore, the inhibition degree of compound on cell proliferation can be monitored by fluorescence value.

### 1.2 Experimental steps

### 1.2.1 Preparation of concentration of compound working solution

1) The compound was diluted 2 mM with 100% DMSO, that is, 5µL of 10 mM compound storage solution was taken and put into the first column of 96-well compound plate, then 20µL of 100% DMSO were added and mixed well;
2) 20µL of 100% DMSO was added to the second column to the tenth column of 96-well compound plate;
3) 5µL of compound was taken from the first column to the second column and mixed well, and then 5µL of compound was taken from the second column to the third column and mixed well. The above steps were repeated to the ninth column to complete the gradient dilution of the compound;
4) Preparation of compound working solution: 2µL of compound per well from the second column of the compound plate was taken to a new 96 well compound plate, and 78µL of cell culture medium per well was added; 2µL of 100% DMSO from the negative control well was taken and 78µL of cell culture medium was added; and
5) The compound concentration was the final concentration, in which the blank control well was the culture medium well without cells, and the negative control was 0.5% DMSO.

### 1.2.2 Cell inoculation and drug treatment

1) The BCR-FMS-BaF3 cell suspension was collected and centrifuged at 1000 rpm (revolutions per minute) for 5 minutes. The supernatant was removed, cells were resuspended with preheated medium and counted. After counting, the cell suspension was diluted with cell culture medium to the required density. The cell density of each type was shown in table 4-1. Each well was inoculated with 80µL cell suspension into 96 well cell culture plate.
2) On the day of the experiment, 20µL of compound working solution was added to the cell plate and incubated for 3 days at 37°C in a 5% CO₂ incubator. The incubation time of different cells was different, and the specific cell density and incubation days were shown in Table 8.

**Table 8 Plating density of cells and co-incubation time of cells and compounds**

| **Cells name** | **Plating density (cells/well)** | **Compound incubation days (days)** |
|---|---|---|
| BCR-FMS-BaF3 | 10000 | 3 |

3) After the incubation is completed, 20µL of CTG detection reagent was added to each well of the cell plate, and incubated at 25°C for 10 minutes. After the incubation is completed, EnVision was used for luminescence signal detection.

### 1.3 Data analysis

The cell survival rate after treatment with compound was calculated using the following formula: % inhibition rate = (RFU_{compound} - RFU_{negative control})/(RFU_{blank} - RFU_{negative control})×100%. Blank reading value: Average reading value of blank control well; Negative control: Cells were treated with 0.5% DMSO, and Prism was used to plot and calculate the IC₅₀ value of the compound.

### 1.4 Experimental results: See Table 9.

**Table 9 Inhibition of cell proliferation by crystal form B of compound of formula (II)**

| **Cells** | **Compound** | **IC₅₀ (nM)** | | |
|---|---|---|---|---|
| | | N=1 | N=2 | Mean value±SD |
| **BCR-FMS-BaF3** | **Crystal form B of Compound of Formula (II)** | 2.46 | 2.47 | 2.46±0.008 |

| | | | | |
|---|---|---|---|---|
| **Experimental conclusion:** Crystal form B of compound of formula (II) can significantly inhibit the proliferation of BCR-FMS-BaF3 cells. | | | | |

## Claims

1. A crystal form A of a compound of formula (I), wherein the X-ray powder diffraction pattern thereof has characteristic diffraction peaks at 2Θ diffraction angles of 6.12±0.20°, 9.25±0.20°, and 14.45±0.20°,

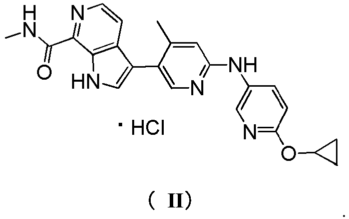

2. The crystal form A according to claim 1, wherein the X-ray powder diffraction pattern thereof has characteristic diffraction peaks at 2Θ diffraction angles of 6.12 ± 0.20°, 7.98 ± 0.20°, 9.25 ± 0.20°, 14.45 ± 0.20°, 16.02 ± 0.20°, and 24.52 ± 0.20°.

3. The crystal form A according to claim 2, wherein the X-ray powder diffraction pattern thereof has characteristic diffraction peaks at 2Θ diffraction angles of 6.12±0.20°, 7.98±0.20°, 9.25±0.20°, 12.21±0.20°, 14.45±0.20°, 16.02±0.20°, 20.20±0.20°, and 24.52±0.20°.

4. The crystal form A according to claim 3, wherein the X-ray powder diffraction pattern thereof has characteristic diffraction peaks at 2Θ diffraction angles of 6.12±0.20°, 7.98±0.20°, 9.25±0.20°, 10.08±0.20°, 12.21±0.20°, 14.45±0.20°, 15.13±0.20°, 16.02±0.20°, 20.20±0.20°, 21.35±0.20°, 21.96±0.20°, and 24.52±0.20°.

5. The crystal form A according to claim 4, wherein the X-ray powder diffraction pattern thereof has characteristic diffraction peaks at 2Θ diffraction angles of 6.12°, 7.98°, 9.25°, 10.08°, 12.21°, 14.45°, 15.13°, 16.02°, 18.34°, 20.20°, 21.35°, 21.96°, 23.41°, 24.52°, 25.43°, 27.39°, 28.56°, 29.03°, 29.96°, 31.75°, and 37.38°.

6. The crystal form A according to claim 5, wherein the crystal form A has an X-ray powder diffraction pattern substantially as shown in Figure 1.

7. The crystal form A according to any one of claims 1-6, wherein the crystal form A has a differential scanning calorimetry curve that has an endothermic peak with an onset of 200.9±3.0°C.

8. The crystal form A according to claim 7, wherein the crystal form A has a differential scanning calorimetry curve as shown in Figure 2.

9. A compound of formula (II),

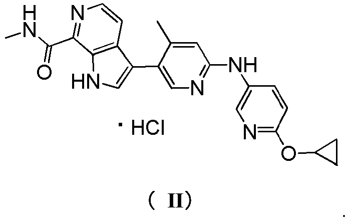

10. A crystal form B of the compound of formula (II), wherein the X-ray powder diffraction pattern thereof has characteristic diffraction peaks at 2Θ diffraction angles of 4.579±0.200°, 6.898±0.200°, and 15.918±0.200°,

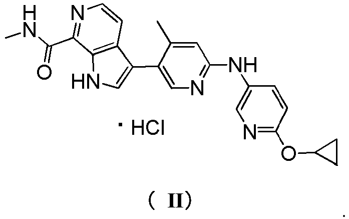

11. The crystal form B according to claim 10, wherein the X-ray powder diffraction pattern thereof has characteristic diffraction peaks at 2Θ diffraction angles of 4.579±0.200°, 6.898±0.200°, 13.019±0.200°, 15.918±0.200°, 19.697±0.200°, and 25.200±0.200°.

12. The crystal form B according to claim 11, wherein the X-ray powder diffraction pattern thereof has characteristic diffraction peaks at 2Θ diffraction angles of 4.579±0.200°, 6.898±0.200°, 7.941±0.200°, 11.218±0.200°, 13.019±0.200°, 15.918±0.200°, 19.697±0.200°, and 25.200±0.200°.

13. The crystal form B according to claim 12, wherein the X-ray powder diffraction pattern thereof has characteristic diffraction peaks at 2Θ diffraction angles of 4.579±0.200°, 6.898±0.200°, 7.941±0.200°, 11.218±0.200°, 13.019±0.200°, 14.701±0.200°, 15.918±0.200°, 17.338±0.200°, 19.697±0.200°, 22.540±0.200°, 25.200±0.200°, and 27.798±0.200°.

14. The crystal form B according to claim 13, wherein the X-ray powder diffraction pattern thereof has characteristic diffraction peaks at 2Θ diffraction angles of 4.579°, 6.898°, 7.941°, 9.070°, 9.423°, 11.218°, 13.019°, 13.796°, 14.701°, 15.182°, 15.918°, 17.338°, 18.939°, 19.461°, 19.697°, 20.700°, 21.602°, 22.200°, 22.540°, 22.818°, 23.919°, 24.461°, 25.200°, 25.639°, 26.563°, 27.120°, 27.798°, 28.156°, 28.595°, 29.000°, 29.339°, 30.739°, 32.064°, 32.355°, 33.201°, 34.156°, 35.020°, 36.063°, and 39.176°.

15. The crystal form B according to claim 14, wherein the crystal form B has an X-ray powder diffraction pattern substantially as shown in Figure 3.

16. The crystal form B according to any one of claims 10-15, wherein the crystal form B has a differential scanning calorimetry curve with an endothermic peak with an onset of 240.16±3.0°C and an exothermic peak having a peak top at 282.04±3.0°C.

17. The crystal form B according to claim 16, wherein the crystal form B has a differential scanning calorimetry curve as shown in Figure 4.

18. The crystal form B according to any one of claims 10-15, wherein the crystal form B has a weight loss of 0.3643% at 200.0 ± 3°C in its thermo gravimetric analysis curve.

19. The crystal form B according to claim 18, wherein the crystal form B has a thermo gravimetric analysis curve as shown in Figure 5.

20. Use of the crystal form A according to any one of claims 1-8, the compound according to claim 9 or the crystal form B according to any one of claims 10-19 in the preparation of a medicament related for treating tenosynovial giant cell tumor.
